# EUROPEAN PATENT APPLICATION

(11) **EP 3 427 777 A1**
(43) Date of publication of application: **16.01.2019**
(21) Application number: 18182613.2
(22) Date of filing: 10.07.2018
(51) Int. Cl.: A61M 5/142, A61M 5/31, A61M 1/00, A61M 39/00

(54) **MEDICAL INJECTION SUCTION DEVICE**

(30) Priority: 11.07.2017 TW 10610195 U
(71) Applicant: Benepet Co., Ltd., Taipei City 10489 (TW)
(72) Inventor: Tan, Ta-Lun, Taipei City (TW)
(74) Representative: Swindell & Pearson Limited

(57) **Abstract**

The present invention relates to a medical injection suction device, comprising: a housing containing a driving motor therein; a syringe mount defined by a wall of an upper side of the housing; a plurality of valves disposed on the wall of the upper side of the housing and on a side of the syringe mount; a slider disposed on a lateral side of the housing and configured to move toward or away from the housing; a driving rod connected to the slider and extending through a wall of the lateral side of the housing, wherein an end of the driving rod is drivingly connected to the driving motor, and the driving motor is configured to drive the driving rod to move the slider; and at least one slide rod, wherein an end of the at least one slide rod is connected to the wall of the lateral side of the housing, and wherein the slider is slidably disposed on the slide rod. The present invention has the advantages of quantitative precision, stable infusion, automatic operation, long service life and prevention of cross-infection.

## Description

### TECHNICAL FIELD

The present invention relates to a medical injection suction device, in particular, to a medical injection suction device capable of automatically and stably and precisely injecting and drawing medical infusions or waste fluids.

### BACKGROUND

In the medical field, medical personnel often have to manually perform injection or suction in a steady way when suctioning waste fluids (lung water, ascites, pleural effusion, pericardial effusion, or waste dialysate from dialysis) from the body or injecting medical infusions (drips, blood, or dialysate). When performed manually, however, the injection of medical infusions or the suction of waste fluids can be performed neither precisely nor at a steady rate. when the patient is physically sensitive (for example, when the patient is a small animal), the amount of injection or suction must be very precise, and the rate of injection or suction must be very slow and steady; otherwise, it is likely to cause harm to the patient. Thus, it not only is unsafe but also requires a lot of manpower in the case of manual performance by a medical person.

Therefore, it is necessary to develop a medical injection suction device to solve the above problems.

### SUMMARY

An objective of the present invention is to provide a medical injection suction device, which (1) can precisely quantify the injection amount of medical infusions and the suction amount of waste fluids; (2) can inject medical infusions in a slow but continuous and stable manner; (3) has the function of preventing catheter backflow or blockage, and can automatically inject infusions without a medical person's personal performing and monitoring, saving a lot of manpower; (4) has a stable and safe structure and prolonged product life; and (5) has the function of preventing cross-infection resulting from repeated use of consumables used in conjunction therewith.

The technical solution adopted by the present invention for solving the above technical problems is as follows:

A medical injection suction device, comprising:
a housing containing a driving motor therein;
a syringe mount defined by a wall of an upper side of the housing;
a plurality of valves disposed on the wall of the upper side of the housing and on a side of the syringe mount;
a slider disposed on a lateral side of the housing and configured to move toward or away from the housing;
a driving rod connected to the slider and extending through a wall of the lateral side of the housing, wherein an end of the driving rod is drivingly connected to the driving motor, and the driving motor is configured to drive the driving rod to move the slider; and
at least one slide rod, wherein an end of the at least one slide rod is connected to the wall of the lateral side of the housing, and wherein the slider is slidably disposed on the slide rod.

In an embodiment of the present invention, the syringe mount is configured to fix a syringe to the housing. The plurality of valves are configured to close and open a plurality of catheters connected to the syringe. The slider is configured to be connected to a plunger of the syringe, and configured to push and pull the plunger of the syringe to inject and suction liquids.

In an embodiment of the present invention, the slider is not disposed on the upper side of the housing.

In an embodiment of the present invention, the medical injection suction device further comprises a stop block disposed at an end of the slide rod and configured to stop the slider from moving off the slide rod.

In an embodiment of the present invention, the medical injection suction device further comprises a control device configured to be electrically connected to the driving motor and control the driving of the driving motor to move the slider and configured to be electrically connected to the plurality of valves and control the closing and opening of the valves.

In an embodiment of the present invention, the medical injection suction device further comprises a flow sensor configured to sense a flow rate and a flow direction of a liquid in a catheter.

In an embodiment of the present invention, the control device is electrically connected to the flow sensor and receives a signal of the flow rate and the flow direction sensed by the flow sensor, wherein the control device stops the movement of the slider and issues an alarm in response to the flow rate being lower than a predetermined value, or the control device stops the movement of the slider and issues an alarm in response to the flow direction not being a predetermined direction.

In an embodiment of the present invention, the medical injection suction device further comprises a displacement sensing device including a resistance ruler, wherein the displacement sensing device reads a difference in resistance value of the resistance ruler and thereby measures a displacement of the slider.

In an embodiment of the present invention, the control device is electrically connected to the displacement sensing device and receives a signal of the displacement of the slider measured by the displacement sensing device, wherein the control device stops the movement of the slider in response to the displacement of the slider measured by the displacement sensing device reaching a predetermined displacement so as to execute a predetermined injection amount or a predetermined suction amount.

In an embodiment of the present invention, the control device comprises a timing unit configured to calculate and measure time, and the control device drives the driving motor for a predetermined amount of time for injection or a predetermined amount of time for suction to complete the predetermined displacement of the slider so as to execute a predetermined injection rate or a predetermined suction rate.

In an embodiment of the present invention, the medical injection suction device further comprises a barcode scanner electrically connected to the control device, wherein the barcode scanner sends a start signal to the control device in response to the completion of scanning of a correct barcode, and wherein the control device cannot be started until the start signal is received.

In an embodiment of the present invention, the medical injection suction device further comprises a control device, wherein the control device comprises an input interface, an output interface and a memory, configured respectively to input a patient data, output the patient data and store the patient data.

In an embodiment of the present invention, the medical injection suction device further comprises a control device comprising a transceiving unit for uploading the patient data or an alarm in the control unit to a cloud terminal, a remote computer or a mobile device.

The technical advantages of the medical injection suction device of the present invention are as follows:
(1) The plurality of valves are configured to close and open a plurality of catheters connected to the syringe. The slider is configured to be connected to a plunger of the syringe, and configured to push and pull the plunger of the syringe to inject and suction liquids. During a suction, a first valve is opened to keep a first catheter conductive; the slider moves a specific distance away from the housing, thereby pulling the plunger of the syringe to draw a liquid (infusion or waste fluid) into the syringe. The displacement sensing device senses the displacement of the slider to precisely quantify the infusion amount of the medical infusion and the suction amount of the waste fluid, wherein the displacement sensing device measures the displacement of the slider by means of a difference in resistance values of the resistance ruler, rather than by using a conventional grating, increasing the precision of quantification. During an injection, the first valve is closed to close the first catheter and a second valve is opened to keep a second catheter conductive; the slider moves a specific distance toward the housing, thereby pushing the plunger of the syringe to inject or expel a fluid (infusion or waste fluid).
(2) The medical injection suction device of the present invention provides the slow but steady injection of a liquid of the syringe into a patient's body at a specific rate by applying a steady force to the plunger of the syringe in order to overcome the problem of instability during injection or suction resulting from pushing the fluid in the catheter traditionally by manually pushing the plunger of the syringe or by gravity.
(3) In the medical injection suction device of the present invention, when the flow sensor detects that the flow rate in the catheter is too low or the flow is not in the predetermined direction, the control device stops moving to push or pull the syringe plunger and sends an alarm to a medical person. Therefore, the medical injection suction device of the present invention prevents catheter backflow or blockage and does not require the personal operation and monitoring of medical personnel, thereby saving a lot of manpower.
(4) In the medical injection suction device of the present invention, the slider is disposed on a lateral side of the housing to move toward or away from the housing instead of being provided on an upper side of the housing to move on the wall of the upper side of the housing. Therefore, the rod (for example, a driving rod) connecting the slider to the driving motor does not extend through the wall of the upper side of the housing to form a hole or slit through the wall of the upper side. Instead, it extends through the wall of a lateral side of the housing so that liquid does not flow into the housing through a hole or slit of the wall of the upper side when the liquid leaks from the syringe, damaging the medical injection suction device. By this stable and safe device structure the life of the product is prolonged.
(5) In the medical injection suction device of the present invention, the consumables used in conjunction with the device have a barcode on the package, and the control device can be started into opeartion for a specific amount of time only after the barcode scanner scans the barcode on the package. The barcode cannot be used to start the control device again after being scanned so as to ensure that each time the medical injection suction device of the present invention is used, new, clean consumables are used in conjunction with the device to avoid the repeated use of consumables which leads to cross-infection.

### BRIEF DESCRIPTION OF DRAWINGS

To describe the technical solutions of the prior art and the embodiments of the present invention more clearly, the following brief introduction is given of the drawings that are needed for the description of the embodiments or the prior art. Apparently, the drawings described below are merely some embodiments of the present invention, and a person having ordinary skill in the art may derive other drawings from these drawings without creative efforts.
FIG. 1 is a schematic diagram of the external appearance of the medical injection suction device according to an embodiment of the present invention.
FIG. 2 is a schematic diagram of the internal structures of the medical injection suction device according to an embodiment of the present invention.
FIG. 3 is a schematic diagram of the external appearance of a peritoneal dialysis catheter for animals which can be used in conjunction with the medical injection suction device of an embodiment of the present invention and serves as a comsumable used in conjunction therewith.
FIG. 4 is a flow chart of the use of the peritoneal dialysis device for animals in conjunction with the peritoneal dialysis catheter for animals according to an embodiment of the present invention.

### Reference Numerals

100 Medical injection suction device
110 Housing
111 Driving motor
112 Wall of the upper side
113 Wall of the lateral side
120 Syringe mount
121 Fixing member
131 First valve
132 Second valve
133 Third valve
140 Slider
150 Driving rod
160 Slide rod
161 Stop block
170 Control device
180 Flow sensor
190 Displacement sensor
191 Resistance ruler
192 Guiding rod
193 Probe module
200 Barcode scanner
1 Peritoneal dialysis catheter for animals
10 Dialysate catheter
20 Injection catheter
24 Peritoneal catheter
30 Waste fluid catheter
33 Waste fluid container
40 Syringe catheter
41 Syringe
42 Plunger
50 Four-way connector

### DETAILED DESCRIPTION

The present invention will be further described in detail with reference to the following embodiments. The following embodiments are intended to explain the present invention, but the present invention is not limited to these embodiments.

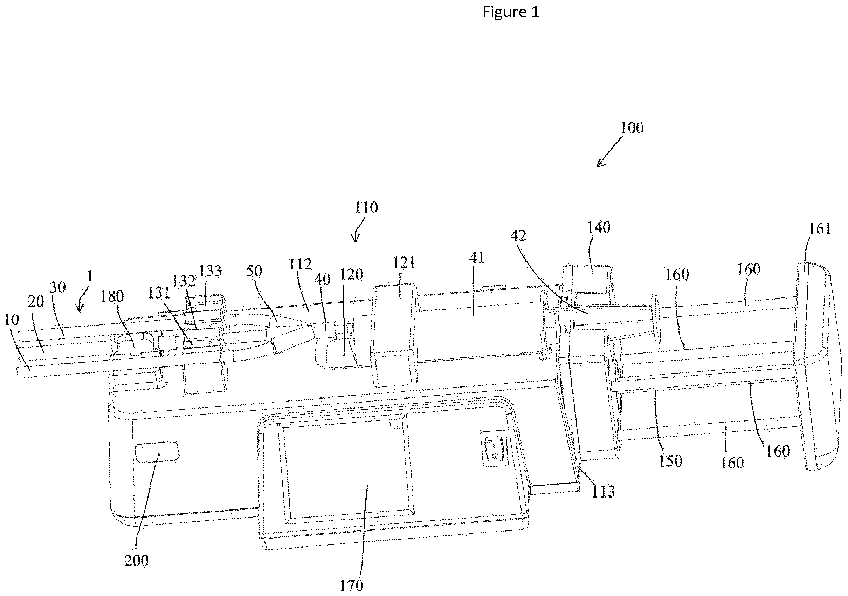

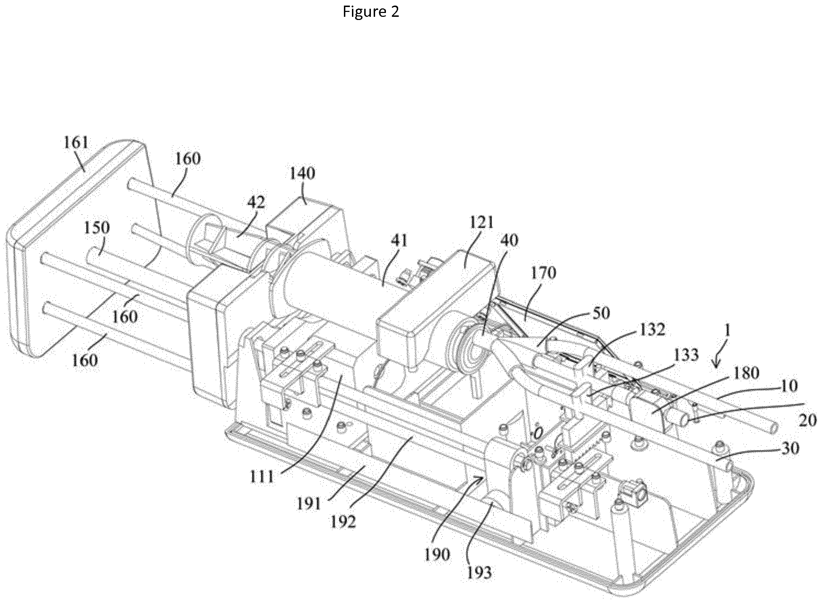

Referring now to FIG. 1 and FIG. 2, FIG. 1 is a schematic diagram of the external appearance of the medical injection suction device according to an embodiment of the present invention, and FIG. 2 is a schematic diagram of the internal structures of the medical injection suction device according to an embodiment of the present invention. In order to achieve the above objective and overcome the drawbacks of the prior art, the present invention provides a medical injection suction device 100 comprising: a housing 110 containing a driving motor 111 therein; a syringe mount 120 defined by a wall 112 of an upper side of the housing 110; a plurality of valves 131, 132, 133 disposed on the wall 112 of the upper side of the housing 110 and on a side of the syringe mount 120; a slider 140 disposed on a lateral side of the housing and configured to move toward or away from the housing 110; a driving rod 150 connected to the slider 140 and extending through a wall 113 of the lateral side of the housing 110, wherein an end of the driving rod 150 is drivingly connected to the driving motor 111, and the driving motor 111 is configured to drive the driving rod 150 to move the slider 140; at least one slide rod 160, wherein an end of the at least one slide rod is connected to the wall 113 of the lateral side of the housing 110, and wherein the slider 140 is slidably disposed on the slide rod 160; a control device 170 configured to be electrically connected to the driving motor 111 and control the driving of the driving motor 111 to move the slider 140 and configured to be electrically connected to the plurality of valves 131, 132, 133 and control the closing and opening of the valves; a flow sensor 180 configured to sense a flow rate and a flow direction of a liquid in a catheter; and a displacement sensing device 190 configured to sense a displacement of the slider 140.

The housing 110 includes a plurality of mechanical components and a plurality of electronic components such as the driving motor 111, a portion of the driving rod 150, the control device 170, the flow sensor 180, the displacement sensor 190, and the like. The syringe mount 120 is configured to fix a syringe 41 to the wall 112 of the upper side of the housing 110. In an embodiment of the present invention, a fixing member 121 may also be provided on the syringe mount 120, as shown in FIG. 1. A spring is provided at the base of the fixing member 121 so that the fixing member 121 clamps and fixes the syringe 41 to the wall 112 of the upper side of the housing 110. This configuration of the fixing member 121 is only an example of its implementation. It may also be implemented as other components such as a snap-fit element, a magnetic fastener or the like to fix the syringe, which should not limit the scope of the present invention.

The present invention provides a medical injection suction device 100 comprising a plurality of valves 131, 132, 133. The number of the valves can be two, three or even more. In the embodiment of the present invention shown in FIG. 1 and FIG. 2, the number of the valves is three, but it is only an example and may also be other numbers, and should not limit the scope of the present invention. The plurality of valves 131, 132, 133 are disposed on the wall 112 of the upper side of the housing 110 and are located on the front end side (the opposite side to the position of the plunger) of the syringe mount 120 and configured to close (shut) and opens (make conductive) a plurality of catheters 10, 20, 30 connected to the syringe. The plurality of catheters 10, 20, 30 respectively can be used for suctioning or injecting infusions and for expelling or suctioning waste fluids. The plurality of valves 131, 132, 133 may be implemented as solenoid valves. The opening or closing of the valves may be controled by the magnetic force generated by energizing/de-energizing the solenoids of the solenoid valves. Therefore, an automatic control of the valves may be realized by electrically connecting the control device 170 to the plurality of valves.

In an embodiment of the present invention, the driving rod 150 is a threaded rod and is connected to the slider 140. The slider 140 rotatably engages the threads on the driving rod 150 through the hole in the slider 140. Further, the driving rod 150 extends through a wall 113 of the lateral side of the housing 110, wherein an end of the driving rod 150 is drivingly connected to the driving motor 111, and the driving motor 111 rotationally drives the driving rod 150 to move the slider 140.

The number of the slide rod 160 is at least one, and may be two, three or even more. In FIG. 1 and FIG. 2, the number of the slide rod 160 is four, passing through the four corners of the slider 140. However, this number of the slide rods is only an example and should not limit the scope of the present invention. An end of the slide rod is connected to the wall 113 of the lateral side of the housing 110, and the slider 140 is slidably disposed on the slide rod 160 to prevent the slider 140 from rotating in the circumferential direction along with the driving rod when the driving rod rotates. In this way, the slider 140 will only move in the direction in which the threads of the driving rod advances (i.e., the direction of the driving rod or the slide rod). As shown in FIG. 1 and FIG. 2, the medical injection suction device further comprises a stop block 161 disposed at an end of the slide rod 160 and configured to stop the slider 140 from moving off the slide rod.

In an embodiment of the present invention, as shown in FIG. 1 and FIG. 2, the slider 140 is provided with a groove configured to engage or fix the plunger 42 of the syringe 41. When the driving motor 111 drives or moves the slider 140, the slider 140 pushes and pulls the plunger of the syringe to inject the liquid in the barrel of the syringe or to draw liquid (such as infusion or waste fluid) into the barrel of the syringe.

A technical feature of the medical injection suction device of the present invention consists in that, the slider 140 is disposed on a lateral side of the housing 110 to move toward or away from the housing 110 instead of being provided on an upper side of the housing 110 to move on a wall 112 of the upper side of the housing. Therefore, the rod (for example, a driving rod) connecting the slider 140 to the driving motor 111 does not extend through the wall 112 of the upper side of the housing to form a hole or slit through the wall 112 of the upper side. Instead, it extends through a wall 113 of a lateral side of the housing. Therefore, another technical feature of the medical injection suction device of the present invention consists in that, the wall 112 of the upper side of the housing does not have openings so that liquid does not flow into the housing 110 through a hole or slit of the wall 112 of the upper side when the liquid leaks from the syringe, damaging the medical injection suction device. By this stable and safe device structure the life of the product is prolonged.

In an embodiment of the present invention, the control device 170 includes a central processing unit (CPU), a memory, a timer, an input interface, and an output interface. The control device 170 is implemented as a microcomputer having a microprocessor as the central processing unit (CPU), wherein a plurality of functions of the central processing unit are integrated into a single chip integrated circuit (IC). The control device 170 may also be implemented as a microcontroller, also referred to as a single-chip microcomputer, which is a type of microcomputer in which a central processing unit, a memory, a timer, and a variety of input and output interfaces are integrated in an integrated circuit chip. The memory is used to store instructions, data, and patient information, including volatile memory such as Dynamic Random Access Memory (DRAM) or Static Random Access Memory (SRAM), as well as non-volatile memory such as Read Only Memory (ROM), flash memory, solid state drive, magnetic drive, and the like. In an embodiment, the input interface and the output interface are a touch-control liquid crystal screen for inputting a patient data and outputting the patient data such as patient name, medical record number, weight, total infusion volume, infusion volume per kg body weight, amount of time for injection, injection rate, amount of time for suction, suction rate, residence time and so on. In an embodiment, the control device 170 further includes a transceiver unit that uploads the patient data in the control unit to a cloud terminal, a remote computer or a mobile device, or sends an alarm to a cloud terminal, a remote computer, or a mobile device to alert medical personnel. In addition, in an embodiment, data from the cloud terminal, the remote computer, or the mobile device may be received by the transceiver unit, for example, to carry out online data update or software update. However, the above control device 170 and the internal components thereof are merely an example and should not limit the scope of the present invention.

The control device 170 is electrically connected to the driving motor 111, the plurality of valves 131, 132, 133, the flow sensor 180, and a displacement sensor 190. The control device 170 controls the driving of the driving motor 111 to move the slider 140 for injection or suction. The control device 170 controls the closing and opening of the valves 131, 132, 133 to select the catheters 10, 20, 30 for injection or suction. The control device 170 receives a signal of the flow rate and the flow direction measured by the flow sensor 180 to monitor the status of the fluid in the injection catheter 20 in real time. The control device 170 receives a signal of the displacement of the slider 140 measured by the displacement sensing device 190 to measure a displacement of the slider 140 and calculates the amount of injected liquid and the amount of suctioned liquid based on the displacement of the slider.

In an embodiment of the present invention, the flow sensor 180 includes a light sensing unit. The fluid in the injection catheter 20 flows through a rotating member and drives the rotating member to rotate. The rotating member rotates to change the frequency of the light received by the light sensing unit, and the rotating speed and the rotating direction of the rotating member are calculated to deduce the flow rate and the flow direction of the fluid. For a specific structure of the flow sensor 180, reference may be made to the prior application of Taiwan Patent Application No. 106113057 by the present inventor. When the flow rate is lower than a predetermined value, it indicates that the catheter 20 may be blocked, and the control device 170 stops the movement of the slider 140, immediately interrupting the injection or suction procedure and sends an alarm to a cloud terminal, a remote computer or a mobile device such as a mobile phone of a medical person via a transceiver device 200. Alternatively, when the flow direction is not a predetermined direction (for example, the flow direction detected is the flow direction of suction while it should have been the flow direction of injection), it indicates that the catheter may be severely blocked, and the control device 170 stops the movement of the slider 140, immediately interrupting the injection or suction procedure and sends an alarm via the transceiver to a cloud terminal, a remote computer, or a mobile device such as a mobile phone of a medical person.

In an embodiment of the present invention, the displacement sensing device 190 includes a probe module 193, a guiding rod 192, and a resistance ruler 191. The displacement sensing device 190 is disposed inside the housing 110. The guiding rod 192 extends through the wall 113 of the lateral side of the housing 110. One end of the guiding rod 192 is connected to the slider 140, and the other end of the guiding rod 192 is connected to the probe module 193; and a resistance ruler 191 is disposed on a side of the probe module 193. The guiding rod 192 is configured to push the probe module 193 to move along the direction of the resistance ruler 191 and the probe module 193 contacts the resistance ruler 191 to read a change of resistance value of the resistance ruler 191, thereby measuring a displacement of the slider 140. In the present embodiment, measuring the displacement of the slider 140 by using the resistance ruler 191 is more precise than measuring the displacement of the slider 140 by using a grating as in the prior art. For a specific structure of the displacement sensing device 190, reference may be made to Taiwan Patent Application No. 106207198 previously filed by the present inventor.

A predetermined injection amount or a predetermined suction amount is input to the control device 170 through the input interface. The control device 170 calculates a predetermined displacement amount of the slider 140 (for example, a forward distance or a backward distance) based on the predetermined injection amount or suction amount. The control device 170 drives the driving motor 111 to move the slider 140. When the displacement of the slider sensed by the displacement sensing device reaches a predetermined displacement, The control device 170 stops the movement of the slider so as to execute the predetermined injection amount or the predetermined suction amount.

A predetermined injection rate or a predetermined suction rate is input to the control device 170 through the input interface. The control device 170 calculates a predetermined amount of time for injection or a predetermined amount of time for suction based on the predetermined injection rate or the predetermined suction rate. The timing unit is used to calculate and measure time. The control device drives the driving motor for the predetermined amount of time for injection or the predetermined amount of time for suction to complete the predetermined displacement of the slider so as to execute the predetermined injection rate or the predetermined suction rate.

In an embodiment of the present invention, the medical injection suction device 100 further comprises a barcode scanner 200 electrically connected to the control device 170, and the consumables used in conjunction with the medical injection suction device 100 have a barcode on the package. Upon scanning the barcode on the package, the barcode scanner 200 sends a start signal to the control device 170. The control device 170 can be started for a specific amount of time (for example, a predetermined amount of time for one treatment) only after receiving the start signal. The barcode cannot be used again to start the control device 170 after being scanned so as to ensure that each time the medical injection suction device 100 is used, new, clean consumables must be used to avoid repeated use of the consumables which leads to cross-infection.

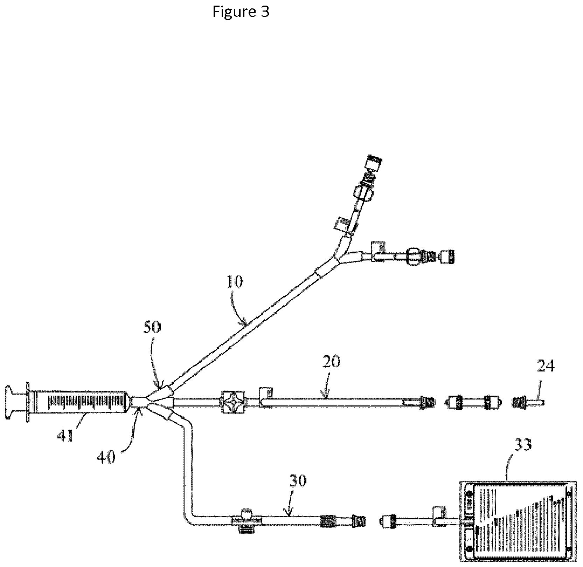

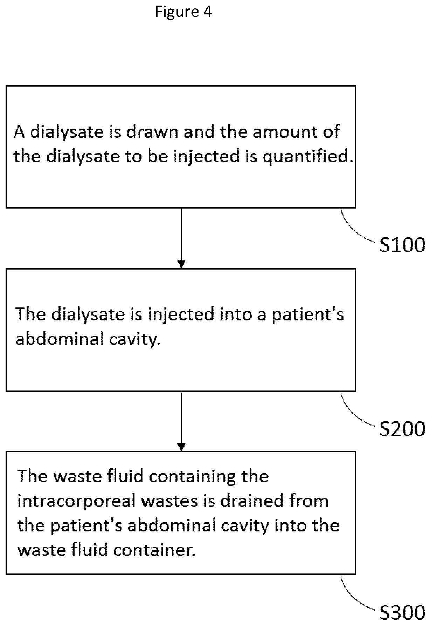

Since the medical injection suction device 100 of the present invention can be used to perform peritoneal dialysis, an embodiment of a method of using the medical injection suction device 100 of the present invention in conjunction with a peritoneal dialysis catheter 1 for animals is provided below. Referring to FIG. 3 and FIG. 4, FIG. 3 is a schematic diagram of the external appearance of a peritoneal dialysis catheter 1 for animals. The peritoneal dialysis catheter 1 for animals can be used in conjunction with the medical injection suction device 100 according to an embodiment of the present invention and is used as a consumable in conjuction therewith. FIG. 4 is a flow chart of the use of the peritoneal dialysis device 100 for animals in conjuction with the peritoneal dialysis catheter 1 for animals according to an embodiment of the present invention.

Step S100: A dialysate is drawn and the amount of the dialysate to be injected is quantified. The medical injection suction device 100 of the present invention opens the first valve 131 to make the dialysate catheter 10 conducting and close the second valve 132 and the third valve 133 to close the injection catheter 20 and the waste fluid catheter 30. The slider 140 moves in a direction away from the housing 110 to pull the plunger 42 of the syringe 41 to generate a negative pressure to draw the dialysate out of a dialysate container. The dialysate flowing out of the dialysate container passes through the dialysate catheter 10, a four-way connector 50 and the syringe catheter 40 sequentially into the syringe 41.

Step S200: The dialysate is injected into a patient's abdominal cavity. The medical injection suction device 100 of the present invention opens the second valve 132 to make the injection catheter 20 conducting and closes the first valve 131 and the third valve 133 to close the dialysate catheter 10 and the waste fluid catheter 30. The slider 140 moves in a direction toward the housing 110, pushing the plunger 42 of the syringe 41 and thereby forcing the dialysate out of the syringe 41. The dialysate flowing from the syringe 41 passes through the syringe catheter 40, the four-way connector 50, the infusion catheter 20, and the peritoneal catheter 24 sequentially into the abdominal cavity of the patient such that the dialysate comes into contact with the patient's peritoneum for a period of time to transfer intracorporeal wastes into the dialysate using the peritoneum as a semipermeable membrane for dialysis.

Step S300: The waste fluid containing the intracorporeal wastes is drained from the patient's abdominal cavity into the waste fluid container 33. The medical injection suction device 100 of the present invention opens the second valve 132 and the third valve 133 to make the infusion catheter 20 and the waste fluid catheter 30 conducting and closes the first valve 131 to close the dialysate catheter 10 and the slider 140 is maintained at a position closest to the housing 110 to maintain the plunger 42 of the syringe 41 in a state of being pushed to the bottom. Thus, the pressure in the abdominal cavity pushes the waste fluid containing the intracorporeal wastes to move toward the waste fluid container 33. The waste fluid flowing from the abdominal cavity passes through the peritoneal catheter 24, the infusion catheter 20, the four-way connector 50 and the waste fluid catheter 30 sequentialy into the waste fluid container 33.

In an embodiment of the present invention, a user may input a procedure of injection and suction via the input interface (liquid crystal touch screen). Taking the above peritoneal dialysis for example, in step S100, the input may be opening the first valve 131, closing the second valve 132 and the third valve 133, and drawing a quantified dialysate at a predetermined rate (or for a predetermined amount of time). In step S200: the input may be opening the second valve 132, closing the first valve 131 and the third valve 133, and injecting the quantified dialysate at a predetermined rate for a residence time of 50 minutes. In step S300: the input may be opening the third valve 133, closing the first valve 131 and the second valve 132, draining the waste fluid for 10 minutes, and repeating the steps S100 to S300 a predetermined number of times. The control device 170 then automatically executes the input injection and suction procedure. In an embodiment of the present invention, the user even only needs to select a modular treatment program via the input interface (liquid crystal touch screen). The control device 170 then automatically executes all the input injection and suction procedure. Taking the above peritoneal dialysis for example, the user only needs to select "peritoneal dialysis" among the options of the treatment program and input the relevant background information of the patient, such as body weight. The control device 170 then calculates an appropriate injection amount of the dialysate, an injection time, a residence time, a waste fluid discharge time based on the body weight, and executes the appropriate injection and suction procedure.

To summarize, in the medical injection suction device 100 of the present invention, (1) the plurality of valves 131, 132, 133 are configured to close and open the plurality of catheters connected to the syringe, and the slider 140 is configured to be connected to the plunger 42 of the syringe 41 and to push and pull the plunger 42 of the syringe 41 to inject and suction the liquid, and the amount of the injected infusion and the amount of the suctioned waste fluid are precisely quantified by using the displacement sensing device 190 to sense the displacement of the slider 140, wherein the displacement sensing device 190 measures the displacement of the slider using the sensed resistance value of the resistance ruler 191 instead of using a conventional grating, improving the precision of quantification; (2) the medical injection suction device 100 of the present invention provides the slow but steady injection of a liquid in the syringe 41 into a patient's body at a specific rate by applying a steady force to the plunger 42 of the syringe 41 in order to overcome the problem of instability during injection resulting from pushing the fluid in the catheter traditionally by manually pushing the plunger 42 of the syringe 41 or by gravity. (3) the flow sensor 180 detects the flow rate and the flow direction in the catheter 20 to prevent backflow or blockage in the catheter 20, eliminating the need for personal execution and monitoring of the medical personnel, thereby saving a lot of manpower; (4) the slider 140 is disposed on a lateral side of the housing 110 and does not extend through the wall of the upper side of the housing 110 to form a hole or slit in the wall 112 of the upper side, so that the leaked liquid does not pass through the hole or slit of the wall 112 of the upper side into the housing 110, resulting in damage to the medical injection suction device 100 and a prolonged product life; and (5) the consumables used in conjunction with the medical injection suction device 100 have a barcode on the package, and the control device 170 can be only after the barcode scanner 200 scans the barcode on the package so as to ensure that each time the medical injection suction device 100 of the present invention is used, new, clean consumables are used in conjunction with the device to avoid the repeated use of consumables which leads to cross-infection.

In conlusion, the embodiments of the present invention are disclosed as above, but the above embodiments are not intended to limit the present invention. Those of ordinary skill in the art may make various alterations and modifications without departing from the spirit and scope of the present disclosure. Therefore, the protection scope of the present invention should be defined by the claims.

## Claims

1. A medical injection suction device, comprising:
a housing containing a driving motor therein;
a syringe mount defined by a wall of an upper side of the housing;
a plurality of valves disposed on the wall of the upper side of the housing and on a side of the syringe mount;
a slider disposed on a lateral side of the housing and configured to move toward or away from the housing;
a driving rod connected to the slider and extending through a wall of the lateral side of the housing, wherein an end of the driving rod is drivingly connected to the driving motor, and the driving motor is configured to drive the driving rod to move the slider; and
at least one slide rod, wherein an end of the at least one slide rod is connected to the wall of the lateral side of the housing, and wherein the slider is slidably disposed on the slide rod.

2. The medical injection suction device according to claim 1, wherein the syringe mount is configured to fix a syringe to the housing, wherein the plurality of valves are configured to close and open a plurality of catheters connected to the syringe, wherein the slider is configured to be connected to a plunger of the syringe and to push and pull the plunger of the syringe to inject and suction liquids.

3. The medical injection suction device according to claim 1, wherein the slider is not disposed on the upper side of the housing.

4. The medical injection suction device according to claim 1, further comprising: a stop block disposed at an end of the slide rod and configured to stop the slider from moving off the slide rod.

5. The medical injection suction device according to claim 1, further comprising: a control device configured to be electrically connected to the driving motor and control the driving of the driving motor to move the slider and configured to be electrically connected to the plurality of valves and control the closing and opening of the valves.

6. The medical injection suction device according to claim 5, further comprising: a flow sensor configured to sense a flow velocity and a flow direction of a liquid in a catheter;

7. The medical injection suction device according to claim 6, wherein the control device is electrically connected to the flow sensor and receives a signal of the flow velocity and the flow direction sensed by the flow sensor, wherein the control device stops the movement of the slider and issues an alarm in response to the flow velocity being lower than a predetermined value, or the control device stops the movement of the slider and issues an alarm in response to the flow direction not being a predetermined direction.

8. The medical injection suction device according to claim 5, further comprising: a displacement sensing device comprising a resistance ruler, wherein the displacement sensing device reads a difference of resistance value of the resistance ruler to measure a displacement of the slider.

9. The medical injection suction device according to claim 8, wherein the control device is electrically connected to the displacement sensing device and receives a signal of the displacement of the slider measured by the displacement sensing device, wherein the control device stops the movement of the slider in response to the displacement of the slider measured by the displacement sensing device reaching a predetermined displacement so as to execute a predetermined injection amount or a predetermined suction amount.

10. The medical injection suction device according to claim 9, wherein the control device comprises a timing unit configured to calculate and measure time, and wherein the control device drives the driving motor for a predetermined amount of time for injection or a predetermined amount of time for suction to complete the predetermined displacement of the slider so as to execute a predetermined injection rate or a predetermined suction rate.

11. The medical injection suction device according to claim 5, further comprising: a barcode scanner electrically connected to the control device, wherein the barcode scanner sends a start signal to the control device in response to the completion of scanning of a correct barcode, and wherein the control device cannot be started until the start signal is received.

12. The medical injection suction device according to claim 1, further comprising: a control device, wherein the control device comprises an input interface, an output interface and a memory, configured respectively to input a patient data, output the patient data and store the patient data.

13. The medical injection suction device according to claim 1, further comprising: a control device comprising a transceiving unit for uploading the patient data or an alarm in the control unit to a cloud terminal, a remote computer or a mobile device.
